(19) Europäisches Patentamt
European Patent Office
Office européen des brevets

(11) Publication number: 0 008 884
B1

(12) # EUROPEAN PATENT SPECIFICATION

(45) Date of publication of patent specification: 17.08.83

(21) Application number: 79301618.9

(22) Date of filing: 10.08.79

(51) Int. Cl.³: C 07 D 498/04,
A 61 K 31/42,
A 61 K 31/43,
A 61 K 31/545
//(C07D498/04, 263/00,
205/00), (A61K31/43, 31/42),
(A61K31/545, 31/42)

(54) **Derivatives of clavulanic acid, a process for their preparation and their compositions.**

(30) Priority: 09.09.78 GB 3626578

(43) Date of publication of application:
19.03.80 Bulletin 80/6

(45) Publication of the grant of the patent:
17.08.83 Bulletin 83/33

(84) Designated Contracting States:
CH DE FR GB IT NL

(56) References cited:
DE - A - 2 646 003
DE - A - 2 817 085

Burger, Medicinal Chemistry, New York 1970, p. 70—71

(73) Proprietor: BEECHAM GROUP PLC
Beecham House Great West Road
Brentford Middlesex (GB)

(72) Inventor: Stirling, Irene
80 Woodcrest Walk
Reigate, Surrey (GB)
Inventor: Clarke, Brian Peter
"Coneybury" Drive Spur, Forest Drive
Kingswood Surrey (GB)

(74) Representative: Hesketh, Alan, Dr. et al,
European Patent Attorney Beecham
Pharmaceuticals Great Burgh Yew Tree Bottom
Road
Epsom, Surrey, KT18 5XQ (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 008 884 B1

Courier Press, Leamington Spa, England

Derivatives of clavulanic acid, a process for their preparation and their compositions

DE—A—2646003 discloses 9-N-disubstituted amino deoxyclavulanic acid derivatives. Our earlier cognate British Patent Application Number 16764/77—37072/77—50229/77—53866/77 (and also French Patent Application Number 78—11 851, DE—A—28 17 085 and Japan Patent Application Number 48292/78) disclosed inter alia the compounds of the formula (I):

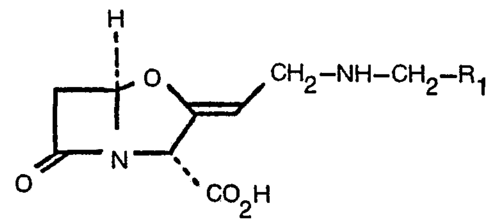

(I)

wherein $R_1$ is a hydrogen atom, an alkyl group of up to 5 carbon atoms, a cycloalkyl group of 5 or 6 carbon atoms, a hydroxyalkyl group of up to 5 carbon atoms or a moiety of the sub-formula (a):

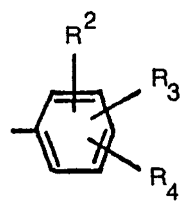

(a)

wherein $R_2$ is a hydrogen, fluorine, chlorine or bromine atom or an alkyl group of 1—3 carbon atoms, an alkoxyl group of 1—3 carbon atoms, an acyloxyl group of 1—3 atoms, a hydroxyl group, an alkoxycarbonyl group containing 1—3 carbon atoms in the alkoxy part, or a group $—N(R_5)CO.R_6$, $—N(R_5)SO_2R_6$ or $—CO—NR_5R_6$ where $R_5$ is a hydrogen atom or an alkyl group of 1—3 carbon atoms or a phenyl or benzyl group and $R_6$ is an alkyl group of 1—3 carbon atoms or a phenyl or benzyl group; $R_3$ is a hydrogen, fluorine or chlorine atom or an alkyl group of 1—3 carbon atoms, an alkoxyl group of 1—3 carbon atoms or an acyloxyl group of 1—3 carbon atoms; and $R_4$ is a hydrogen fluorine or chlorine atom or an alkyl group of 1—3 carbon atoms or an alkoxyl group of 1—3 carbon atoms.

It has been found that other 9-aralkylaminodeoxyclavulanic acid derivatives may be prepared that have antibacterial and $\beta$-lactamase inhibitory properties.

Accordingly the present invention provides the compounds of the formula (II):

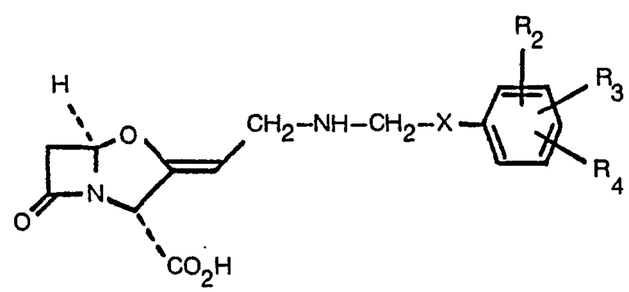

(II)

or an ester thereof wherein $R_2$, $R_3$ and $R_4$ are as defined in relation to formula (I) and X is an alkylene group of 1 to 4 carbon atoms.

The compounds of the formula (II) per se exist in the form of zwitterions, that is they may be represented as shown in formula (IIa):

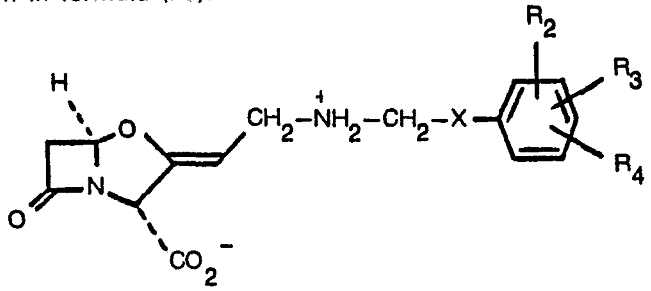

(IIa)

if desired wherein $R_1$ is as defined in relation to formula (II). These zwitterionic compounds form a favoured aspect of this invention in view of their generally crystalline form.

The esters of the compounds of the formula (II) may be presented in the form of the free base or in the form of an acid addition salt.

Suitably $R_2$ is a hydrogen, fluorine or chlorine atom or a methoxyl, ethoxyl, hydroxyl, acetoxyl, propionyloxy, methyl, ethyl, methoxycarbonyl, ethoxy-carbonyl or acetamido group.

Suitably $R_3$ is a hydrogen, fluorine or chlorine atom or a methoxyl, ethoxyl, acetoxyl, propionoxyl, methyl or ethyl group.

Suitably $R_4$ is a hydrogen, fluorine or chlorine atom or a methoxyl, ethoxyl, acetoxyl, propionoxyl methyl or ethyl group.

More suitably $R_2$ is a hydrogen, fluorine or chlorine atom or a methoxyl, hydroxyl, methyl or acetamido group.

More suitably $R_3$ is a hydrogen, fluorine or chlorine atom or a methoxyl or methyl group.

More suitably $R_4$ is a hydrogen atom or a methyl or methoxyl group.

Preferably $R_2$ is a hydrogen, fluorine or chlorine atom or a methyl, methoxyl or acetamido group.

Preferably $R_3$ is a hydrogen atom or methoxyl group.

Preferably $R_4$ is a hydrogen atom.

Suitably X is a straight chained alkylene group of 1 to 4 carbon atoms. Suitably X is an alkylene group of 1 to 3 carbon atoms branched to carry a methyl group.

More suitably X is a $CH_2$ or $CH_2.CH$ group.

As has been previously indicated we prefer to prepare and use the crystalline zwitterionic compounds within the formula (II). However, esters of the compounds of the formula (II) also form part of this invention, for example as the free base or as the acid addition salt, since such compounds may also be used to enhance the effectiveness of penicillins or cephalosporins.

Certain suitable esters of the compounds of the formula (II) include those of the formula (III) and (IV):

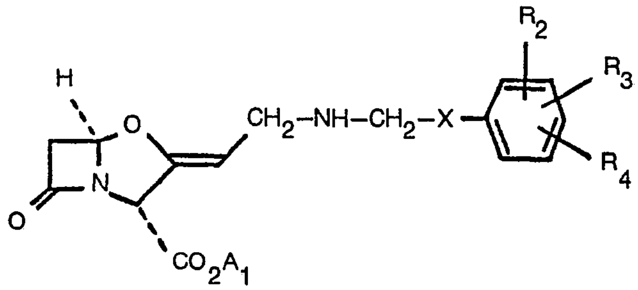

(III)

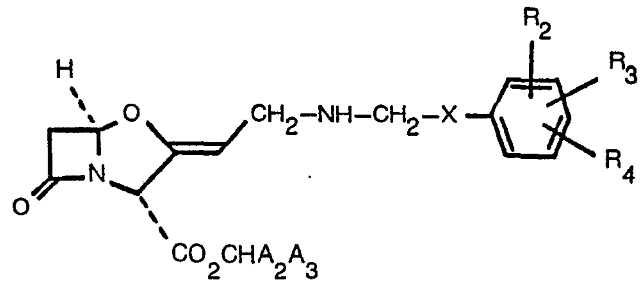

(IV)

wherein X, $R_2$, $R_3$ and $R_4$ are as defined in relation to formula (II) wherein $A_1$ is an alkyl group of 1—6 carbon atoms optionally substituted by an alkoxyl or acyloxyl group of 1—7 carbon atoms; $A_2$ is an alkenyl or alkynyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxyl of up to 4 carbon atoms.

Suitable esters of the compounds of the formula (II) include the methyl, ethyl, n-propyl, n-butyl, allyl, $CH_2—C{\equiv}CH$, methoxymethyl, acetoxymethyl, propionoxymethyl, pivaloyloxymethyl, ethoxycarbonyloxymethyl, methoxycarbonyloxyethyl, ethoxycarbonyloxyethyl, dimethoxyphthalidyl, benzyl, methoxybenzyl, ethoxybenzyl, nitrobenzyl, chlorobenzyl or the like ester.

Certain favoured groups $A_1$ include the methyl, methoxymethyl, acetoxymethyl, acetoxyethyl, phthalidyl, ethoxycarbonyloxymethyl, $\alpha$-ethoxycarbonyloxyethyl and the like groups.

Certain favoured groups $A_2$ include the phenyl and 4-methoxyphenyl groups. A particularly favoured moiety $A_3$ is the hydrogen atom.

Certain other favoured values for $A_1$ include those of the sub-formulae (c), (d) and (e):

$—CHA_5—OA_6$ (c)

$—CHA_5—COA_6$ (d)

$—CHA_5—CO_2A_6$ (e)

wherein $A_5$ is a hydrogen atom or a methyl group and $A_6$ is an alkyl group of up to 4 carbon atoms or a phenyl or benzyl group either of which may be substituted by one or two alkyl or alkoxyl group of up to 3 carbon atoms or by a fluorine, chlorine or bromine atom or a nitro group; or $A_5$ is joined to $A_6$ to form the residue of an unsubstituted saturated 5- or 6-membered heteroalicyclic ring or an ortho-phenylene group which may be substituted by one or two alkyl or alkoxyl groups of up to 3 carbon atoms or by a fluorine, chlorine or bromine atom or nitro group.

An apt acylic value for the sub-group of the formula (c) is $—CH_2—OA_6$.

An apt acylic value for the sub-group of the formula (d) is $—CH_2—CO—A_6$.

An apt acylic value for the sub-group of the formula (e) is $—CH_2—CO_2A_6$.

A further apt acylic value for the sub-group of the formula (e) is $—CH(CH_3)—CO_2A_6$.

Favoured values for $A_6$ in the preceding acylic moieties include the methyl, ethyl, propyl, butyl, phenyl and benzyl groups.

Apt cyclic values for the sub-group of the formula (c) include the tetrahydropyranyl and tetrahydrofuranyl groups.

Esters of the compounds of the formula (II) may be presented in the form of their acid addition salts if desired. The acid used to form the salt will most suitably be pharmaceutically acceptable, but non-pharmaceutically acceptable acid addition salts are also evisaged, for example as intermediates in preparation of the pharmaceutically acceptable salts by ion exchange. Suitable pharmaceutically acceptable acid addition salts include those of inorganic and organic acids, such as hydrochloric, phosphoric, sulphuric, methanesulphonic, toluenesulphonic, citric, malic, acetic, lactic, tartaric, propionic, succinic or the like acid. Most suitably the acid addition salt is provided as a solid and preferably as a crystalline solid.

Compounds of this invention wherein crystalline form may be solvated, for example hydrated.

The present invention provides a pharmaceutical composition which comprise a compound of this invention and a pharmaceutically acceptable carrier.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form. The zwitterionic compounds of this invention are particularly suitable for use in such compositions.

The injectable solution of the compound of this invention may be made up in a sterile pyrogen-free liquid such as water, aqueous ethanol or the like.

Compounds of this invention when in highly pure crystalline form tend to have relatively low aqueous solubilities so that if it is desired to administer substantial quantities of the medicament this can require fairly large quantities of water for reconstitution. In these circumstances it is often convenient to administer the solution intravenously.

An alternative approach to administering the compounds of this invention and especially those zwitterionic compounds of the formula (IIa) is to utilise an injectable suspension. Such suspensions may be made up in sterile water; sterile saline or the like and may also contain suspending agents such as polyvinylpyrrolidone, lecithin or the like (for example in the manner described for amoxycillin trihydrate in Belgian Patent No. 839109). Alternatively such compositions may be prepared in an acceptable oily suspending agent such as acharis oil or its equivalent. The use of suspensions can give rise to advantageously prolonged blood levels of the medicament. Belgian Patent No. 839109 may be consulted for suitable methods and materials for producing injectable aqueous suspensions. For use in such suspensions the zwitterionic compound of this invention should be in the form of fine particles as described in said Belgian Patent.

Unit dose compositions comprising a compound of this invention adapted for oral administration form a further suitable composition aspect of this invention. However, orally administrable forms are generally less favoured than injectable forms owing to the relatively poor absorption of the compounds from the gastro-intestinal tract. Despite this orally administrable compositions are of use as a

synergistically effective blood level can be expected at high doses and at lower doses such compositions may be used to treat infections localised in the gastro-intestinal tract.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin since the resulting composition shows enhanced effectivness (synergy).

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, celbenicillin, and other known penicillins including prodrugs therefore such as their in-vivo hydrolysable esters such as the acetoxymethyl, pivaloyloxymethyl, $\alpha$-ethoxycarbonyloxyethyl or phthalidyl esters of ampicillin, benzylpenicillin or amoxycillin, and aldehyde or ketone adducts of penicillins containing an 6-$\alpha$-aminoacetamide side chain (such as hetacillin, metapicillin and analogous derivatives of amoxycillin) or $\alpha$-esters of carbenicillin or ticarcillin such as their phenyl or idanyl $\alpha$-esters.

Suitable cephalosporins for inclusion in the compositions of this invention include cefatrizine, cephaloridine, cephalothin, cefazolin, cephalexin, cephacetrile, cephamandole nafate, cephapirin, cepradine, 4-hydroxycephalexin, cefaparole, cephaloglycin, and other known cephalosporins or prodrugs thereof.

Such compounds are frequently used in the form of a salt or hydrate or the like.

Naturally if the penicillin or cephalosporin present in the composition is not suitable for oral administration then the composition will be adapted for parenteral administration. As previously indicated such injectable or infusible compositions are preferred.

Highly favoured penicillins for use in the compositions of this invention include ampicillin, ampoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin. Such cephalosporins may be used as a pharmaceutically acceptable salt, for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent). Orally administrable compositions containing a compound of the invention will normally contain relatively more synergist than corresponding injectable compositions, for example the ratio in an oral composition may be from about 3:1 to about 1:1 whereas a corresponding injectable composition may contain a ratio of about 1:1 to about 1:3 (compound of the invention: penicillin or cephalosporin).

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of inter alia, the respiratory tract, the urinary tract and soft tissues in humans and mastitis in cattle.

Normally between 50 and 1000 mg of the compounds of the invention will be administered each day of treatment but more usually between 100 and 750 mg of the compounds of the invention will be administered per day, for example as 1—6 doses, more usually as 2, 3 or 4 doses.

The penicillin or cephalosporin in the synergistic composition of this invention will normally be present at approximately the amount at which it is conveniently used which will usually be expected to be from about 62.5 to 1000 mg per dose, more usually about 125, 250 or 500 mg per dose.

One particularly favoured composition of this invention will contain from 150 to 1000 mg of amoxycillin as the trihydrate or sodium salt and from 25 to 500 mg of a compound of this invention.

Most suitably this form of composition will contain a compound of the formula (IIa).

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin hetacillin, pivampicillinhydrochloride, bacampicillin hydrochloride, or talampicillin hydrochloride. Most suitably this form of the composition will contain a compound of the formula (IIa).

Most suitably the preceding compositions will contain from 200 to 700 mg of the penicillin

component. Most suitably the preceding composition will comprise from 50 to 250 mg of a compound of the formula (IIa) preferably (III) in crystalline form.

Such compositions may be adapted for oral or parenteral use except when containing an in-vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefore and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of the formula (IIa) preferably in crystalline form. Such compositions containing di-salts of carbenicillin and ticarcillin will be adapted for parenteral administration.

The present invention also provides a method of treating bacterial infections in humans or domestic mammals which comprises the administration of a composition of this invention.

Commonly the infection treated will be due to a strain of *Staphylococcus aureus, Klebsiella aerogenes, Escherichia coli, Proteus sp.* or the like. The organisms believed to be most readily treated by an antibacterially effective amount of a compound of this invention is *Staphylococcus aureus.* The other organisms named are more readily treated by using a synergistically effective amount of the compound of the invention and a penicillin or cephalosporin. The administration of the two components may take place separately but in general it we prefer to use a composition containing both the synergist and the penicillin or cephalosporin.

The indications for treatment include respiratory tract and urinary tract infections in humans and mastitis in cattle.

The present invention also provides a process for the preparation of a compound of the formula (II) as hereinbefore defined or an ester thereof which process comprises the hydrogenation of a compound of the formula (V):

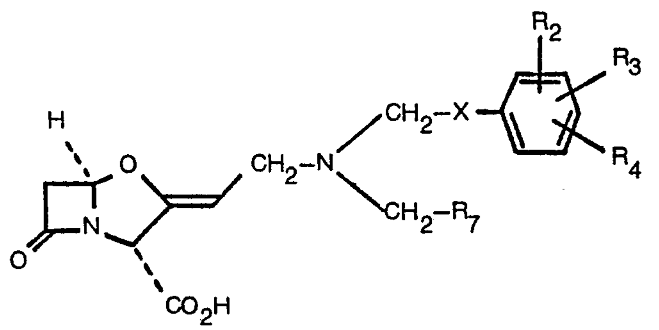

(V)

or an ester thereof wherein X, $R_2$, $R_3$ and $R_4$ are as defined in relation to formula (II) and $R_7$ is a group of the sub-formula (a) or (b):

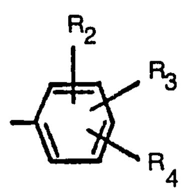

(a)

$$—C(R_8)=CR_9R_{10}$$ (b)

wherein $R_2$, $R_3$ and $R_4$ are as defined in relation to formula (II), $R_8$ is a lower alkyl group, $R_9$ is a hydrogen atom, a lower alkyl or phenyl group and $R_{10}$ is a hydrogen atom, lower alkyl or phenyl group; and thereafter is desired esterifying the zwitterion of the formula (IIa) as hereinbefore defined which was produced by the hydrogenation of the compound of the formula (V) or a hydrogenolysable ester thereof.

Most suitably the group of the sub-formula (a) is the phenyl group.

Most suitably the group of the sub-formula (b) is of the formula $C(CH_3)=CH_2$.

When used herein the term "hydrogenolysable ester" means an ester which on hydrogenation is cleaved to yield the parent carboxylic acid.

The hydrogenation is normally carried out in the presence of a transition metal catalyst.

The catalyst we have preferred to use is palladium, for example in the form of palladium on carbon (charcoal), palladium on barium sulphate, palladium on calcium carbonate or the like.

A favoured catalyst is palladium on carbon (sometimes referred to as palladium on charcoal); for example 5%, 10%, 20% or 30% palladium on carbon.

A low, medium or high pressure of hydrogen may be used in this reaction, for example from 1 to 6 atmospheres.

The reaction is normally carried out at a non-extreme temperature, for example from 0°C to 30°C and more usually from 12°C to 25°C. It is generally convenient to carry out the reaction at ambient temperature.

Suitably solvents for carrying out the hydrogenation include ethanol, n-propanol, isopropanol, tetrahydrofuran, dioxane, ethyl acetate or mixtures of such solvents or such solvents in the presence of water. A favoured solvent is ethanol.

We have preferred to carry out the hydrogenation reaction on a hydrogenolysable ester of a compound of the formula (V) wherein $R_7$ is a $C(CH_3){=}CH_2$ group so that a compound of the formula (II) per se is formed by the hydrogenation. Such hydrogenation reactions proceeds at least in part via the formation of a compound of the formula (V). Favoured hydrogenolysable esters include benzyl and substituted benzyl esters such as methoxybenzyl, nitrobenzyl (for example the p-nitrobenzyl ester), chlorobenzyl, bromobenzyl and like esters. A particularly suitable hydrogenolysable ester is the benzyl ester. A further particularly suitable hydrogenolysable ester is the p-methoxybenzyl ester.

The product may generally be isolated from the reaction mixture by filtering off the solids (the catalyst which should be well washed to remove the product) and then evaporating the solvent, preferably under low pressure, to yield the initial product. Further purification may be effected by such conventional methods as chromatography over cellulose or other mild stationary phase eluting with a $C_{1-4}$ alkanol optionally in the presence of water and optionally in the presence of tetrahydrofuran. Evaporation of the combined active fraction (identified by aqueous potassium permanganate spray on tlc) then yields the desired compound in pure form. The desired product is normally obtained in crystalline form (unless it is an unsalted ester). Trituration under ethanol, isopropanol or the like $C_{1-4}$ alkanol or other conventional solvent such as a ketone, ether or ester solvent or other conventional solvent (for example of up to 6 carbon atoms and more suitably of up to 4 carbon atoms) may also be used to aid crystallization. Recrystallisation from ethanol or the like may also be employed. The solvent used in such processes may advantageously be moist.

Unsalted esters of the compounds of the formula (II) tend to be oils so that it is often more convenient for handling to convert them into solid acid addition salts, for example by reaction with one equivalent of an acid. Alternatively the non-hydrogenolysable ester of the compound of the formula (V) may be hydrogenated in the presence of one equivalent of an acid, that is they may be hydrogenated in the form of their acid addition salt.

The compounds of the formula (II) may be hydrogenated in the form of their acid addition salts with a strong acid but this is not a preferred form of the process of this invention.

The present invention also provides a process for the preparation of an ester of a compound of the formula (II) which process comprises the reaction of the compound of the formula (II) with an esterifying agent.

The zwitterionic compound of the formula (II) may be dissolved or suspended in a solvent such as dimethylformamide, hexamethylphosphoramide, dichloromethane, ethyl acetate or other non-esterifiable solvents and therein esterified. Suitable temperatures for such a reaction range from about 0° to about 25°C. Suitable esterifying reagents include reactive halides and their equivalents, alkyl oxonium salts and the like.

When a reagent such as a reactive iodide, chloride, bromide, tosylate, mesylate or the equivalent is used, the resulting salt is generally suitable for use in a composition of this invention. Alternatively, the salt may be converted to a free base or alternative salt. When an alkyl oxonium salt is used, it is preferred to convert the resulting tetrafluoroborate to the free base or alternative salt. The various aforementioned salts may be converted to the free base by neutralisation, for example by contacting a solution of the salt in water with an organic phase, neutralising the salt by adding a base and extracting the liberated amine into the organic phase. This amine may thereafter be re-salted by reacting with an appropriate acid, for example in a dry organic solvent. It is generally preferred to use not more than one equivalent of acid for this process. Alternatively, the originally formed salt may be converted into the alternative salt using an ion exchange material, for example, by passing an aqueous solution of one salt through a bed of an anion exchange resin in the form of the desired salt such as the chloride form.

The salts may normally be obtained in solid form by dissolving in a fairly polar organic solvent (such as ethanol, tetrahydrofuran or the like) and then precipitating using a non-polar solvent such as diethyl ether, cyclohexane or the like.

The salts of the esters of the compounds of the formula (II) may normally be obtained in crystalline form by conventional methods such as trituration under (or crystallisation or recrystallisation from) a suitable organic solvent such as ether, acetone, acetonitrile, tetrahydrofuran or the like.

The present invention also provides a process for the preparation of an ester of the compound of the formula (II) which process comprises the reaction of an acid addition salt of the compound of the formula (II) with an alcohol in the presence of a condensation promoting agent.

Suitable condensation promoting agents for use in this process include carbodiimides such as dicyclohexylcarboxiimide and the chemical equivalents thereof.

The acid addition salt may be formed in situ or may be preformed. The acid employed will

normally be a strong acid such as a methane sulphonic acid, p-toluene sulphonic or the like or trifluoroacetic acid or the like.

The reaction is normally carried out in an inert organic solvent. When the ester being formed is that of a liquid alcohol it is convenient to use that alcohol as the solvent or as part of the solvent system. The esterification is generally performed at a non-extreme temperature such as 0° to 35°C, for example from about 10° to 25°C. Conveniently the reaction mixture may be performed at ambient temperature.

Other methods of preparing esters of the compounds of the formula (II) are those described in the aforementioned patents and applications which are incorporated herein by reference.

The following Examples illustrate the invention.

Example 1

Benzyl 9-N-(2-methylalyl)-N-(3-trans phenylallyl)aminodeoxyclavulanate

Benzyl dichloroacetyl clavulanate (5.9g; 14.8mm) in dimethylformamide (40 $cm^3$) at 0° was treated with *N*-(2-methylallyl-*N*-(3-*trans* phenylallyl) amine (1.9 equivalents) and stirred at 0° for $1\frac{3}{4}$ hours. The mixture was then poured into ethyl acetate (250 $cm^3$) and washed with water (5 × 100 $cm^3$) and saturated brine (5 × 100 $cm^3$), dried (anhydrous magnesium sulphate) and evaporated to an oil. This oil was chromatographed on silica gel eluting with ethylacetate—cyclohexane (1:2), fractions were collected containing the title compound; Rf ($SiO_2$/ethylacetate:cyclohexane; 1:1) = 0.76, detection by aqueous potassium permanganate spray. Combined fractions were evaporated to yield an oil, 3.49 g (51%). $\nu$ (film) 1805, 1750, 1700, 1495, 1450, 1305, 1230, 1175, 1120, 1080, 1045, 1015, 967, 895, 745, 695 $cm^{-1}$. $\delta$ ($CDCl_3$) 1.71 (3H, s, $CH_2C(CH_3)$=$(CH_2)$, 2.89 (2H, s, $NCH_2C(CH_3)$=$CH_2$, 2.93 (1H, d, *J* 7Hz, 6$\beta$C*H*), 3.07 (2H, d, *J* 6Hz, $NCH_2CH$=CH), 3.16 (2H, d, *J* 7Hz, $9CH_2$), 3.39 (1H, dd, *J* 17 and 3Hz, 6$\alpha$C*H*), 4.72 (1H, t, *J* 7Hz, 8C*H*), 4.75—4.95 (2H, broad m, $CH_2C(CH_3)$=$CH_2$), 50.6 (1H, s, 3C*H*), 5.16 (2H, s, $CO_2CH_2C_6H_5$), 5.58 (1H, d, *J* 3Hz, 5$\alpha$C*H*), 6.15 (1H, dt, *J* 16 and 6Hz, $NCH_2CH$=CH—), 6.45 (1H, d, *J* 16 Hz, $NCH_2$—CH=C*H*—), 7.15—7.50 (10H, m, 2 × $C_6H_5$).

Example 2

9-N-(3-phenylpropyl)aminodeoxyclavulanic acid

Benzyl 9-*N*-(2-methylallyl-*N*-(3-*trans*-phenylallyl aminodeoxyclavulanate (1:1 g; 2.4 mm) in ethanol (40$cm^3$ was hydrogenolysed for 20 minutes at atmospheric pressure in the presence of palladium on carbon (10% Pd; 0.3g; prehydrogenated for 20 minutes). The catalyst was filtered off and washed with ethanol (20 $cm^3$), then washed separately with aqueous ethanol (100 $cm^3$), this aqueous washing was evaporated to a crystalline solid. This solid was chromatographed on cellulose eluting with butanol-propan-2-ol-water; 4:4:1. Fractions were collected containing the title compound, Rf ($SiO_2$/butanol-propan-2-ol-water; 7:7:6) = 0.64 (detection by aqueous potassium permanganate spray) Combined fractions were evaporated to yield a crystalline solid, this solid was washed with cold ethanol and dried to yield 100 mg (13%) of the title compound.

$\nu$(NUJOL) 3350 (broad), 1803, 1695, 1615, 1565, 1305, 1290, 1185, 1125, 1090, 1050, 1020, 1005, 925, 895, 760 750, 725, 695 $cm^{-1}$. $\delta$($D_2O$/DMSO(20%) 1.72—2.10 (2H, m, $\overset{+}{N}H_2CH_2CH_2CH_2$—), 2.57—3.02 (4H, m, $\overset{+}{N}H_2CH_2CH_2CH_2C_6H_5$), 3.00 (1H, d, *J* 17 Hz, 6$\beta$C*H*), 3.52 (1H, dd, *J* 17 and 3Hz, 6$\alpha$C*H*), 3.62 (2H, s, *J* 7Hz, $9CH_2$), 8C*H* obscured by HOD, 4.90 (1H, s, 3C*H*), 5.67 (1H, d, *J* 3Hz, 5$\alpha$C*H*), 7.23 (5H, s, $C_6H_5$).

Example 3

Composition

a) The compound of Example 2 (50 mg) in sterile form may be dissolved in water for injection B.P. (2.5 ml) to form an injectable solution.

b) The compound of Example 2 (50 mg) in sterile form and sodium amoxycillin (150 mg) in sterile form may be dissolved in water for injection B.P. (2.5 ml) to form an injectable solution.

Example 4

Benzyl 9-N(2-methyl-3-phenylallyl-N-(4-phenylbutyl)aminodeoxyclavulanate

Benzyl 9-0 dichloroacetyl clavulanate (6.0 g; 15.1 mmol) in dry dimethylformamide (60 ml) was cooled, with stirring, to −20°C. N-(2-methyl-3-phenylallyl)-N-(4-phenylbutyl)amine (8.0 g; 28.7 mmol) in dry dimethylformamide (80 ml) was then added dropwise over a period of 20 minutes. The reaction mixture was then allowed to warm to −10°C and stirring continued for 1 hour. The solution was then poured into an ethyl acetate/water mixture and shaken. The two layers were separated and the aqueous layer extracted with more ethyl acetate. The combined organic layers were washed with a saturated solution of sodium chloride, dried ($MgSO_4$) and evaporated to an oil. Column chromatography afforded the title compound as a colourless oil Rf ($SiO_2$/ethyl acetate:petroleum ether; 1:1)=0.6 $\nu_{max}$ ($CHCl_3$): 1800, 1745 and 1695 $cm^{-1}$. $\delta$ ($CDCl_3$): 1.30 to 1.70 (4H, m, $CH_2CH_2CH_2Ph$); 1.82 (3H, s, $CH_3$); 2.35 (2H, broad t, *J* 7 Hz, N—$CH_2CH_2CH_2CH_2Ph$); 2.57 (2H, broad t, *J* 7 Hz, N—$CH_2CH_2CH_2CH_2Ph$); 2.89

(1H, d, *J* 17 Hz, 6β-C*H*); 2.93 (2H, s, N—$CH_2$—C=); 3.16 (2H, d, *J* 7 Hz, 9—$CH_2$); 3.36 (1H, dd, *J* 17 and 3 Hz, 6α-C*H*); 4.70 (1H, broad t, *J* 7 Hz, 8—C*H*); 5.05 (1H, s, 3—C*H*); 5.13 (2H, s, $CO_2CH_2$); 5.55 (1H, d, *J* 3 Hz, 5—C*H*); 6.34 (1H, s, $CHC_6H_5$); 7.00 to 7.48 (15H, m, aromatic *H*'s).

Example 5

9-N-(4-Phenylbutyl)aminodeoxyclavulanic acid

Benzyl 9-N-(2-methyl-3-phenylallyl)-N-(4-phenylbutyl)-aminodeoxyclavulanate (1.5 g; 2.7 mmol) in 5% tetrahydrofuran/ethanol (20 ml) was carefully added to a pre-hydrogenated mixture of 10% palladium on charcoal (750 mg) in ethanol (50 ml). The mixture was then hydrogenated at 1 atmosphere until the uptake of hydrogen ceased (1.5 hours). The catalyst was then filtered off through a celite pad and the 'cake' washed well with 75% aqueous ethanol. The ethanol plus washings were then evaporated to dryness to give a yellow solid which on trituration with ethanol gave the title compound as a white crystalline solid (56%).

$\nu_{max}$ (KBr): 1800, 1685 and 1620 (broad) $cm^{-1}$. δ ($D_2O/D_6$ acetone: 1.65—2.15 (4H, m, N—$CH_2CH_2CH_2CH_2$Ph), 2.82 and 3.20 (4H, 2 x m, N—$CH_2CH_2CH_2CH_2$Ph), 3.18 (1H, d, 6β-C*H*, partially obscured by 9—$CH_2$), 3.85 (2H, d, *J* 7 Hz, 9—$CH_2$), 4.85 (1H, br.t, 8—C*H*, partially obscured by HOD), 5.05 (1H, m, 3—C*H*), 5.95 (1H, d, *J* 3 Hz, 5—C*H*) and 7.40 (5H, s, $C_6H_5$).

Example 6

Benzyl 9-N-[2'-(3,4-dimethoxyphenyl) ethyl]-N-(2-methyl-3-phenylallyl)aminodeoxyclavulanate

Benzyl dichloroacetylclavulanate (4.74 g; 11.8 mmol) in dry dimethylformamide (50 $cm^3$) at −10° was treated with 1.9 equivalents of N-[β-(3,4-dimethoxyphenyl) ethyl]-N-(2-methyl-3-phenyl allyl) amine, slowly in dimethylformamide (20$cm^3$), and stirred for 1 hour between −10° and +6°. The mixture was poured into ethylacetate (300$cm^3$) and was washed with water (4 x 200$cm^3$) and saturated brine (5 x 200$cm^3$), dried (anhydrous magnesium sulphate) and evaporated in the presence of toluene to low volume, this crude product was chromatographed on silica eluting with ethyl acetate-cyclohexane (1:2). Fraction were collected containing the title compound, Rf ($SiO_2$/ethylacetate-cyclohexane; 1:1) = 0.63 (detection by aqueous potassium permanganate spray). Combined fractions were evaporated to afford the title compound as an oil; 2.0g (29%) ν (film) 1805, 1750, 1695, 1515, 1450, 1305, 1265, 1238, 1160, 1030, 745, 700 $cm^{-1}$. δ ($CDCl_3$) 1.83 (3H, s, C($CH_3$)=CH), 2.65 (4H, bs, $NCH_2CH_2$), 2.93 (1H, d, *J* 17 Hz, 6βC*H* partially obscurred), 3.05 (2H, s, $NCH_2C(CH_3)$1), 3.28 (2H, d, *J* 7Hz, 8$CH_2$ partially obscurred), 3.40 (1H, dd, *J*17 and 3Hz, 6α *CH*, partially obscurred), 3.78 and 4.22 (6H, 2 x s, 2 x $OCH_3$), 4.75 (1H, bt, *J* 7Hz, 8C*H*), 5.08 (1H, bs, 3C*H*), 5.16 (2H, s, $CH_2CH_2C_6H_5$), 5.62 (1H, d, *J* 3Hz, 5αC*H*), 6.38 (1H, bs, C($CH_3$)=C*H*), 6.60—6.85 (3H, m, $CH_2C_6H_3$ $(OCH_3)_2$), 7.10—7.50 (10H, m, 2 x $C_6H_5$).

Example 7

9-N-[2-(3,4-Dimethoxyphenyl)ethyl]-aminodeoxyclavulanic acid

Benzyl 9-N-[2-(3,4-dimethoxyphenyl)ethyl]-N-(2-methyl-3-phenylallyl)aminodeoxyclavulanate (1.88g; 3.23 mmol) in ethanol (35 $cm^3$) tetrahydrofuran (12 $cm^3$) and water (5 $cm^3$) was hydrogenolysed at atmospheric pressure in the presence of 10% palladium on carbon (0.62 g; which had been prehydrogenated for 10 minutes) for 35 minutes. The catalyst was filtered off and washed with ethanol (20 $cm^3$) then with aqueous ethanol (200 $cm^3$), this aqueous washing was collected separately and was evaporated to afford a white crystalline solid, this solid was washed with cold ethanol and dried to give the title compound (41%). Rf ($SiO_2$/ethylacetate-ethanol-water; 5:2:2) = 0.54 (detection by aqueous potassium permanganate spray).

ν (Nujol) 1798, 1700, 1635, 1610, 1570, 1510 $cm^{-1}$, ν (KBr) 1972, 1697, 1620, 1515 $cm^{-1}$.

Example 8

Benzyl 9-N-(2-phenylethyl)-N-(2-methyl-3-phenylallyl)aminodeoxyclavulanate

Benzyl dichloracetylavulanate (8g; 20mM) in dry dimethylformamide (50$cm^3$) at −10° was treated with 1.9 equivalents of N-(2-phenylethyl)-N-(2-methyl-3-phenylallyl) amine in 20 $cm^3$ dimethylformamide and stirred for 3/4 hour between −10° and 0°C. The mixture was poured into ethyl acetate (250 $cm^3$) and washed with water (5 x 100 $cm^3$) and saturated brine (6 x 150 $cm^3$), dried (anhydrous magnesium sulphate) and evaporated in the presence of toluene to small volume. This crude product was chromatographed on silica eluting with ethyl acetate-cyclohexane; 1:2. Fractions were collected containing the title compound Rf ($SiO_2$/ethylacetate-cyclohexane; 1:2) = 0.70 (detection by aqueous potassium permanganate spray). Combined fractions were evaporated to afford the title compound (33%). ν (film) 1805, 1750, 1695, 1495, 1453, 1305 1178, 1015, 750, 702 $cm^{-1}$. δ ($CDCl_3$) 1.79 (3H, s, C($CH_3$) = CH), 2.55—2.90 (4H, m, $NCH_2CH_2C_6H_5$), 2.92 (1H, d, *J* 17Hz, 6βC*H*, partially obscured by $NCH_2$ C ($CH_3$)), 3.27 (2H, d, *J* 7Hz, 9$CH_2$, partially obscured by 6 α-CH), 3.40 (1H, dd, *J* 17 and 3Hz, 6αC*H*, partially obscured by 9$CH_2$), 4.73 (1H, bt, *J* 7Hz, 8C*H*), 5.08 (1H, s, 3C*H*), 5.15 (2H, s, $OCH_2C_6H_5$), 5.62 (1H, d, *J* 3Hz, 5αC*H*), 6.37 (1H, bs, C($CH_3$) = C*H*), 7.0—7.5 (15H, m, 2 x $CH_2C_6H_5$, = CH —$C_6H_5$).

Example 9

9-N-(2-Phenylethyl)aminodeoxyclavulanic acid

Benzyl 9-N-(2-phenylethyl)-N-(2-methyl-3-phenylallyl)aminodeoxyclavulanate (1.72 g; 3.3mM) in ethanol (35 $cm^3$), tetrahydrofuran (12$cm^3$) and water (5$cm^3$) was hydrogenolysed at atmospheric pressure in the presence of 10% palladium on charcoal (560mg) for 30 minutes. The catalyst was filtered off and washed with ethanol (30$cm^3$) then with aqueous ethanol (150$cm^3$), this aqueous washing was collected separately and was evaporated to afford the title compound as a white solid. This solid was washed with cold ethanol and dried, yield = 0.50 g. The initial catalyst filtrate and ethanolic washings were evaporated to give an oil, ethanol was added and on cooling crystals formed, these were filtered off, washed with cold ethanol and dried to yield 60mg of the title compound. Rf ($S1O_2$/ethylacetate-ethanol-water (5:2:2)) = 0.50. Total yield = 0.56g (56%) $\nu$ (Nujol) 1805, 1695, 1615$cm^{-1}$. $\delta$($D_2O/(CD_3)_2CO$) 2.80—3.60 (5H, broad m, $NH_2CH_2CH_2C_6H_5$ and 6$\beta$—*CH*), 3.66 (1H, dd, *J* 17 and 3 Hz, 6$\alpha$—CH, partially obscured by 9—$CH_2$), 3.84 (2H, d, *J* 8Hz, 9—$CH_2$), 4.72—5.05 (2H, m, 3—*CH* and 8—*CH*), 5.83 (1H, d, *J* 3Hz, 5—*CH*), 7.38 (5H, s, $CH_2C_6H_5$).

Example 10

Benzyl 9-N-(2-methylallyl)-N-($\alpha$-methylcinnamyl)aminodeoxyclavulanate

Benzyl 9-O-dichloroacetyl clavulanate (6.30 g; 15.8 mmol) in acetonitrile (60 mls) at 4°C was treated with dropwise addition of N-(2-methylallyl)-N-($\alpha$-methylcinnamylamine (6 g; 30 mmol) in acetonitrile (60 mls). On final addition the reaction was stirred at 4 to 10°C for 2 hours. The acetonitrile was then removed under a reduced pressure and the resulting oil was dissolved in ethyl acetate. The solution was then washed with water, brine, dried ($MgSO_4$) and evaporated in vacuo. Silica-gel column chromatography afforded the title compound as an oil. $\nu_{max}$ ($CHCl_3$): 1802, 1745, 1695 and 1600 (br) $cm^{-1}$. $\delta$ ($CDCl_3$): 1.70 (3H, s, $C(CH_3)=CH_2$), 1.82 (3H, s, $C(CH_3)=CH$), 2.84 (2H, s, N—$CH_2$), 2.92 (2H, s, N—$CH_2$), 2.90 (1H, d, *J* 17 Hz, 6$\beta$—*CH*), 3.12 (2H, d, *J* 7 Hz, 9—$CH_2$), 3.38 (1H, dd, *J* 17 and 3 Hz, 6$\alpha$—*CH*), 4.70 (1H, br.t, *J* 7 Hz, 8—*CH*), 4.82 (2H, br.s, C=$CH_2$), 5.06 (1H, s, C—3*H*), 5.16 (2H, s, $CO_2CH_2$), 5.60 (1H, d, *J* 3 Hz, 5—*CH*), 6.36 (1H, s, C = $CHC_6H_5$), 7.22 and 7.30 (10H, 2 x s, 2 x $C_6H_5$).

Example 11

9-N-(3-Phenyl-2-methylpropyl)aminodeoxyclavulanic acid

Benzyl-9-N-(2-methylallyl)-N-($\alpha$-methylcinnamyl) aminodeoxyclavulanate (1.0 g; 2 mmol) in ethanol (20 mls) was carefully added to a pre-hydrogenated mixture of 10% palladium on charcoal (300 mg's) in ethanol (30 mls). The mixture was then hydrogenated at atmospheric pressure for 30 minutes. The catalyst was then filtered off and washed well with aqueous ethanol. The filtrate plus washings were then evaporated to dryness under a reduced pressure. Cellulose column chromatography afforded the title compound as a white crystalline solid. $\nu_{max}$ (KBr): 1790, 1692 and 1610 (br) $cm^{-1}$. $\delta$ ($D_2O$): 0.93 (3H, d, *J* 7 Hz, $CH(CH_3)CH_2C_6H_5$), 2.30 (1H, m, $CH_2CH$), 2.58 (2H, d, *J* 7 Hz, $CH_2C_6H_5$), 2.85 (2H, m, $NH_2^+$—$CH_2$), 2.97 (1h, d, *J* 17 Hz, 6$\beta$—*CH*), 3.54 (1H, dd, *J* 17 and 3 Hz, 6$\alpha$—*CH*), 3.62 (2H, d, *J*7 Hz, 9—$CH_2$), 4.92 (1H, s, 3—*CH*), 5.67 (1H, d, *J* 3Hz, 5—*CH*) and 7.25 (5H, m, $CH_2C_6H_5$). (8—*CH* obscured by HOD peak).

**Claims**

1. A compound of the formula (II):

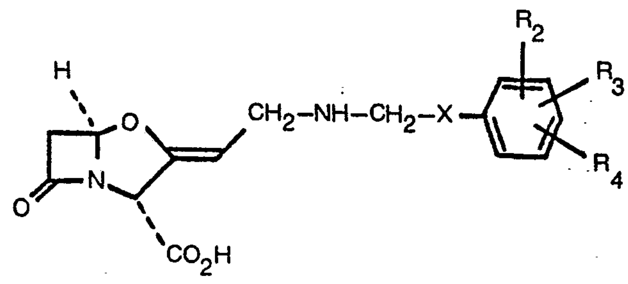

(II)

or an ester thereof wherein $R_2$ is a hydrogen, fluorine, chlorine or bromine atom or an alkyl group of 1—3 carbon atoms, an alkoxyl group of 1—3 carbon atoms, an acyloxyl group of 1—3 atoms, a hydroxyl group, an alkoxycarbonyl group containing 1—3 carbon atoms in the alkoxy part, or a group —$N(R_5)CO.R_6$, —$N(R_5)SO_2R_6$ or —CO—$NR_5R_6$ where $R_5$ is a hydrogen atom or an alkyl group of 1—3 carbon atoms or a phenyl or benzyl group and $R_6$ is an alkyl group of 1—3 carbon atoms or a phenyl or benzyl group; $R_3$ is a hydrogen, fluorine or chlorine atom or an alkyl group of 1—3 carbon atoms, an alkoxyl group of 1—3 carbon atoms or an acyloxyl group of 1—3 carbon atoms; $R_4$ is a hydrogen fluorine or chlorine atom or an alkyl group of 1—3 carbon atoms or an alkoxyl group of 1—3 carbon atoms; and X is an alkylene group of 1—4 carbon atoms.

2. A compound as of the formula (II) as claimed in claim 1 in zwitterionic form.

3. A compound as claimed in claim 2 wherein $R_2$ is a hydrogen, fluorine or chlorine atom or a methoxyl, ethoxyl, hydroxyl, acetoxyl, propionyloxy, methyl, ethyl, methoxycarbonyl, ethoxy-carbonyl or acetamido group; $R_3$ is a hydrogen, fluorine or chlorine atom or a methoxyl, ethoxyl, acetoxyl, propionoxyl, methyl or ethyl group; and $R_4$ is a hydrogen, fluorine or chlorine atom or a methoxyl, ethoxyl, acetoxyl, propionoxyl, methyl or ethyl group.

4. A compound as claimed in claim 3 wherein $R_4$ is a hydrogen atom.

5. A compound as claimed in claims 3 or 4 wherein $R_3$ is a hydrogen atom or a methoxyl group.

6. A compound as claimed in any of claims 3 to 5 wherein $R_2$ is a hydrogen, fluorine or chlorine atom or a methyl, methoxyl or acetamido group.

7. A compound as claimed in any of claims 3 to 6 wherein X is a $CH_2$ or $CH_2CH_2$ group.

8. 9-N-(3-Phenylpropyl)aminodeoxyclavulanic acid.

9. 9-N-(4-Phenylbutyl)aminodeoxyclavulanic acid.

10. 9-N-[2-(3,4-Dimethoxyphenyl)ethyl]aminodeoxyclavulanic acid.

11. 9-N-(2-Phenylethyl)aminodeoxyclavulanic acid.

12. 9-N-(3-Phenyl-2-methylpropyl)aminodeoxyclavulanic acid.

13. A pharmaceutical composition which comprises a compound as claimed in any of claims 1 to 12 and a pharmaceutically acceptable carrier.

14. A composition as claimed in claim 13 which also comprises a penicillin or cephalosporin.

15. A compound as claimed in any of claims 1 to 12 for use as a $\beta$-lactamase inhibitor or antibacterial agent.

16. A process for the preparation of a compound as claimed in any of claims 1 to 12 which process comprises the hydrogenation of a compound of the formula (V):

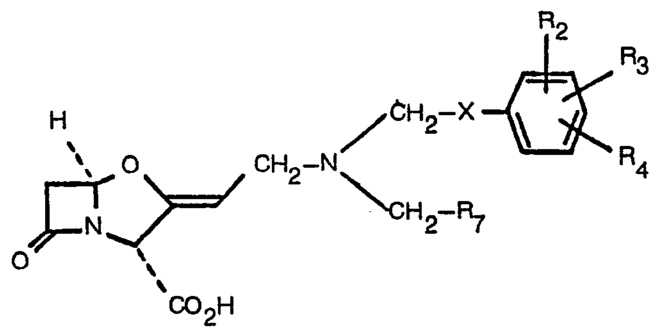

(V)

or an ester thereof wherein X, $R_2$, $R_3$ and $R_4$ are as defined in any of claims 1 to 13 and $R_7$ is a group of the sub-formula (a) or (b):

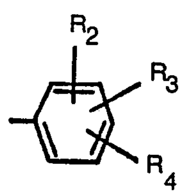

(a)

$-C(R_8)=CR_9R_{10}$ (b)

wherein $R_2$, $R_3$ and $R_4$ are as defined in any of claims 1 to 13; $R_8$ is a lower alkyl group, $R_9$ is a hydrogen atom, a lower alkyl or phenyl group and $R_{10}$ is a hydrogen atom, lower alkyl or phenyl group; and thereafter if desired esterifying the resulting zwitterion.

**Revendications**

1. Composé de formule (II):

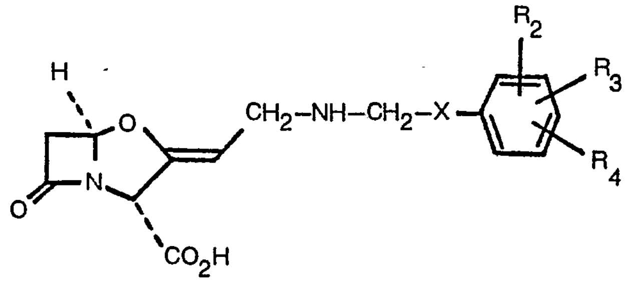

(II)

ou ester de ce composé, formule dans laquelle $R_2$ est un atome d'hydrogène, de fluor, de chlore ou de brome ou un groupe alcoyle ayant de 1 à 3 atomes de carbone, un groupe alcoxy ayant de 1 à 3 atomes de carbone, un groupe acyloxy ayant de 1 à 3 atomes de carbone, un groupe hydroxy, un groupe alcoxycarbonyle contenant de 1 à 3 atomes de carbone dans la fraction alcoxy, ou un groupe —N($R_5$)CO.$R_6$, —N($R_5$(SO$_2$$R_6$ ou —CO—N$R_5$$R_6$, où $R_5$ est un atome d'hydrogène ou un groupe alcoyle ayant de 1 à 3 atomes de carbone, ou bien un groupe phényle ou benzyle, et $R_6$ est un groupe alcoyle ayant de 1 à 3 atomes de carbone ou un groupe phényle ou benzyle; $R_3$ est un atome d'hydrogène, de fluor, ou de chlore ou un groupe alcoyle ayant de 1 à 3 atomes de carbone, un groupe alcoxy ayant de 1 à 3 atomes de carbone, ou un groupe acyloxy ayant de 1 à 3 atomes de carbone; $R_4$ est un atome d'hydrogène, de fluor ou de chlore ou un groupe alcoyle ayant de 1 à 3 atomes de carbone, ou bien un groupe alcoxy ayant de 1 à 3 atomes de carbone; et X est un groupe alcoylène ayant de 1 à 4 atomes de carbone.

2. Composé de formule (II) suivant la revendication 1, sous la forme d'un zwittérion.

3. Composé suivant la revendication 2, dans lequel $R_2$ est un atome d'hydrogène, de fluor ou de chlore ou bien un groupe méthoxy, éthoxy, hydroxy, acétoxy, propionyloxy, méthyle, éthyle, méthoxycarbonyle, éthoxycarbonyle ou acétamido; $R_3$ est un atome d'hydrogène, de fluor ou de chlore ou un groupe méthoxy, éthoxy, acétoxy, propionoxy, méthyle ou éthyle; et $R_4$ est un atome d'hydrogéne, de fluor, ou de chlore ou un groupe méthoxy, éthoxy, acétoxy, propionoxy, méthyle ou éthyle.

4. Composé suivant la revendication 3, dans lequel $R_2$ est un atome d'hydrogène.

5. Composé suivant la revendication 3 ou 4, dans lequel $R_3$ est un atome d'hydrogène ou un groupe méthoxy.

6. Composé suivant l'une quelconque des revendications 3 à 5 dans lequel $R_2$ est un atome d'hydrogène, de fluor ou de chlore ou un groupe méthyle, méthoxy ou acétamido.

7. Composé suivant l'une quelconque des revendications 3 à 6, dans lequel X est un groupe $CH_2$ ou $CH_2CH_2$.

8. Acide 9-N-(3-phénylpropyl)aminodésoxyclavulanique.

9. Acide 9-N-(4-phénylbutyl)aminodésoxyclavulanique.

10. Acide 9-N-[2-(3,4-diméthoxyphényl)éthyl]aminodésoxyclavulanique.

11. Acide 9-N-(2-phényléthyl)aminodésoxyclavulanique.

12. Acide 9-N-(3-phényl-2-méthylpropyl)aminodésoxyclavulanique.

13. Composition pharmaceutique renfermant un composé suivant l'une quelconque des revendications 1 à 12 et un excipient pharmaceutiquement acceptable.

14. Composition suivant la revendication 13, caractérisée en ce qu'elle renferme également une pénicilline ou une céphalosporine.

15. Composé suivant l'une quelconque des revendications 1 à 12, destiné à être utilisé comme inhibiteur de $\beta$-lactamase ou agent antibactérien.

16. Procédé pour la préparation d'un composé suivant l'une quelconque des revendications 1 à 12, ce procédé consistant à effectuer l'hydrogénation d'un composé de formule (V):

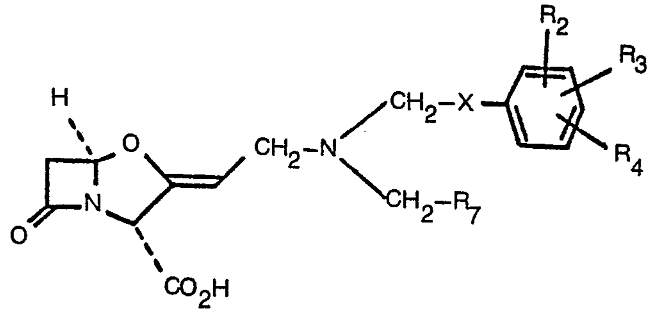

(V)

ou d'un ester de celui-ci, formule dans laquelle X, $R_2$, $R_3$ et $R_4$ sont tels que définis dans l'une quelconque des revendications 1 à 12, et $R_7$ est un groupe de sous-formule (a) ou (b):

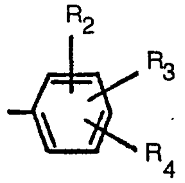

(a)

$$—C(R_8)=CR_9R_{10}$$ (b)

dans lesquelles $R_2$, $R_3$ et $R_4$ sont tels que définis dans l'une quelconque des revendications 1 à 12, $R_8$ est un groupe alcoyle inféfieur, $R_9$ est un atome d'hydrogène ou un groupe alcoyle inférieur ou phényle et $R_{10}$ est un atome d'hydrogène ou un groupe alcoyle inférieur ou phényle, puis, si désiré à estérifier le zwittérion résultant.

**Patentansprüche**

1. Eine Verbindung der Formel (II)

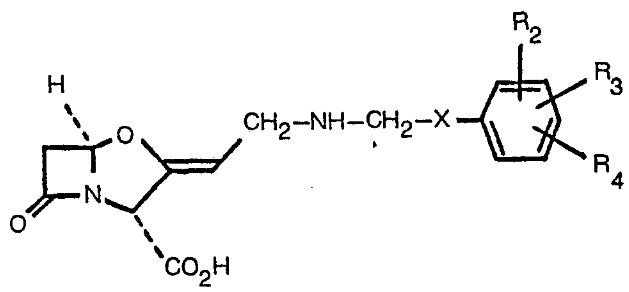

(II)

oder ein Ester derselben, in welcher $R_2$ ein Wasserstoff-, Fluor-, Clor-, oder Bromatom oder eine Alkylgruppe mit 1 bis 3 Kohlenstoffatomen, eine Alkoxygruppe mit 1 bis 3 Kohlenstoffatomen, eine Acyloxygruppe mit 1 bis 3 Kohlenstoffatomen, eine Hydroxylgruppe, eine Alkoxycarbonylgruppe mit 1 bis 3 Kohlenstoffatomen im Alkoxyteil derselben oder eine Gruppe —N $(R_5)CO.R_6$, —$N(R_5)SO_2R_6$ oder —CO—$NR_5R_6$ ist, wobei $R_5$ ein Wasserstoffatom oder eine Alkylgruppe mit 1 bis 3 Kohlenstoffatomen oder eine Phenyl- oder Benzylgruppe und $R_6$ eine Alkylgruppe mit 1 bis 3 Kohlenstoffatomen oder eine Phenyl- oder Benzylgruppe ist; $R_3$ ein Wasserstoff-, Fluor- oder Chloratom oder eine Alkylgruppe mit 1 bis 3 Kohlenstoffatomen, eine Alkoxygruppe mit 1 bis 3 Kohlenstoffatomen oder eine Acyloxygruppe mit 1 bis 3 Kohlenstoffatomen ist; $R_4$ eine Wasserstoff-, Fluor- oder Chloratom oder eine Alkylgruppe mit 1 bis 3 Kohlenstoffatomen oder eine Alkoxygruppe mit 1 bis 3 Kohlenstoffatomen ist und X eine Alkylengruppe mit 1 bis 4 Kohlenstoffatomen ist.

2. Eine Verbindung der Formel (II), wie in Anspruch 1 beansprucht, in Zwitterionenform.

3. Eine Verbindung, wie in Anspruch 2 beansprucht, in welcher $R_2$ ein Wasserstoff-, Fluor- oder Chloratom oder eine Methoxy-, Äthoxy-, Hydroxy-, Acetoxy-, Propionyloxy-, Methyl-, Äthyl-, Methoxycarbonyl-, Äthoxycarbonyl- oder Acetamidogruppe ist; $R_3$ ein Wasserstoff-, Fluor- oder Chloratom oder eine Methoxy-, Äthoxy, Acetoxy-, Propionoxy-, Methyl- oder Äthylgruppe ist; und $R_4$ ein Wasserstoff-, Fluor- oder Chloratom oder eine Methoxy-, Äthoxy-, Acetoxy-, Propionoxy-, Methyl- oder Äthylgruppe ist.

4. Eine Verbindung, wie in Anspruch 3 beansprucht, in welcher $R_4$ ein Wasserstoffatom ist.

5. Eine Verbindung, wie in den Ansprüchen 3 oder 4 beansprucht, in welcher $R_3$ ein Wasserstoffatom oder eine Methoxygruppe ist.

6. Eine Verbindung wie in irgendeinem der Ansprüche 3 bis 5 beansprucht, in welcher $R_2$ ein Wasserstoff-, Fluor- oder Chloratom oder eine Methyl-, Methoxy- oder Acetamidogruppe ist.

7. Eine Verbindung, wie in irgendeinem der Ansprüche 3 bis 6 beansprucht, in welcher X eine $CH_2$- oder $CH_2CH_2$-Gruppe ist.

8. 9-N-(3-Phenylpropyl)aminodeoxyclavulansäure.

9. 9-N-(4-Phenylbutyl)aminodeoxyclavulansäure.

10. 9-N-([2-(3,4-Dimethoxyphenyl)äthylaminodeoxyclavulansäure.

11. 9-N-(2-Phenyläthyl)aminodeoxyclavulansäure.

12. 9-N-(3-Phenyl-2-methylpropyl) aminodeoxyclavulansäure.

13. Eine pharmazeutische Zusammensetzung, welche eine Verbindung, wie in irgendeinem der Ansprüche 1 bis 12 beansprucht, und einen pharmakologisch annehmbaren Träger enthält.

14. Eine Zusammensetzung, wie in Anspruch 13 beansprucht welche außerdem ein Penicillin oder Cephalosporin enthält.

15. Eine Verbindung, wie in irgendeinem der Ansprüche 1 bis 12 beansprucht, zur Verwendung als ein $\beta$-Lactamaseinhibitor oder ein antibakterielles Mittel.

16. Ein Verfahren für die Herstellung einer Verbindung, wie in irgendeinem der Ansprüche 1 bis 12 beansprucht, welches Verfahren die Hydrierung einer Verbindung der Formel (V)

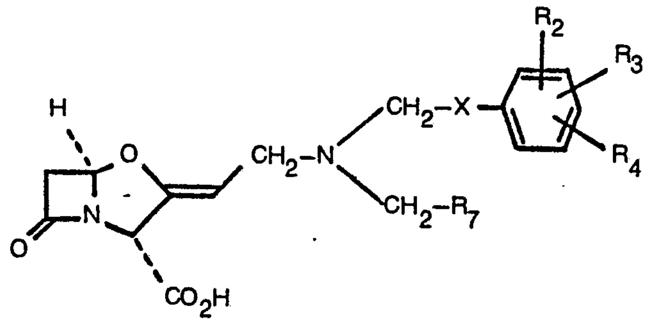

(V)

oder eines Esters derselben, wobei X, $R_2$, $R_3$ und $R_4$ wie in irgendeinem der Ansprüche 1 bis 13 definiert sind und $R_7$ eine Gruppe der Unterformel (a) oder (b) bedeutet:

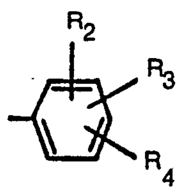

(a)

$$—C—(R_8)=CR_9R_{10} \quad \text{(b)}$$

in denen $R_2$, $R_3$ und $R_4$ wie in irgendeinem der Ansprüche 1 bis 13 definiert sind, $R_8$ eine niedere Alkylgruppe, $R_9$ ein Wasserstoffatom, eine niedere Alkyl- oder eine Phenylgruppe und $R_{10}$ ein Wasserstoffatom, eine niedere Alkyl- oder eine Phenylgruppe bedeutet und gewünschtenfalls eine anschließende Veresterung des gebildeten Zwitterions umfaßt.